# EUROPÄISCHE PATENTSCHRIFT

(11) **EP 0 732 395 B1**
(45) Veröffentlichungstag und Bekanntmachung des Hinweises auf die Patenterteilung: **07.11.2001**
(21) Anmeldenummer: 96250059.1
(22) Anmeldetag: 13.03.1996
(51) Int. Cl.: C12M 3/00, B65G 53/52

(54) **Vorrichtung zur Druckverteilung im Kopfstück einer Apparatur zum ballistischen Transfer von Zellen**
Element for the pressure distribution in th nozzle assembly of an apparatus for the ballistic transfer of cells
Elément pour la distribution de la pression dans l'emboût d'un appareil pour le transfer ballistique de cellules

(30) Priorität: 14.03.1995 DE 19510696
(43) Veröffentlichungstag der Anmeldung: 18.09.1996
(73) Patentinhaber: Soft Gene GmbH, D-14197 Berlin (DE)
(72) Erfinder: Wittig, Burghardt, Prof. Dr., 14197 Berlin (DE); Schroff, Matthias, 30853 Langenhagen (DE); Schroff, Josef, 30853 Langenhagen (DE)
(74) Vertreter: Omsels, Hermann-Josef

(56) Entgegenhaltungen:
- WO-A-91/18991
- DE-A- 3 602 949
- FR-A- 959 484
- FR-A- 2 619 889

## Beschreibung

Die Erfindung betrifft eine Vorrichtung zur Druckverteilung im Kopfstück einer Apparatur zum ballistischen Transfer von Zellen.

Der sogenannte ballistische Transfer von biologischem Material in lebende Zellen verspricht eine wichtige Methode in der Behandlung von Krankheiten mit molekularbiologischen Ansätzen zu werden. Besonders in der klinischen Anwendung kommt der Aspekt des Verfahrens, daß große Zellmengen in einem Arbeitsgang behandelt werden können, vorteilhaft zur Geltung.

Die dem ballistischen Transfer zu Grunde liegende Idee ist in vielfacher Form realisiert und patentiert worden (EP 0 331 855; EP 0 397 413; WO 91/18991; WO 91/00359; WO 91/00915). Zum Markt hat bis heute allerdings nur eine Ausformung der Idee nennenswerten Zugang gefunden. Es handelt sich um ein von der Firma DuPont entwickeltes und geschütztes (WO 91/18991) Gerät, das unter der Bezeichnung "Biolistic" von der Firma Bio-Rad (Hercules, California, USA) weltweit in Lizenz vertrieben wird.

Dieses Gerät arbeitet nach dem Prinzip, durch eine "kalte", d.h. durch Reißen einer Berstscheibe unter Gasdruck eines bei Raumtemperatur befindlichen Gases wie Helium, ausgelöste Gasdruckwelle ein Makroprojektil auf die Zielzellen zu beschleunigen. Das Makroprojektil trägt auf seiner den Zielzellen zugewandten Seite eine Vielzahl von Mikroprojektilen, Körpern, die klein sind im Verhältnis zur Größe der Zielzellen und die auf ihrer Oberfläche adsorbiert die zu transportierende Substanz tragen. Diese Mikroprojektile werden mit dem Makroprojektil beschleunigt und fliegen nach dessen Auftreffen auf ein in der Flugbahn befindliches Rückhaltegitter auf die Zielzellen weiter.

Bei dem beschriebenen Gerät liegt die Zahl der in einem Arbeitsgang zu behandelnden Zellen bei etwa 1 x 10⁶ - 3 x 10⁶. Diese Zahl liegt etwa im Bereich der durch Elektroporation in einem Arbeitsgang zu behandelnden Zellen, doch bietet die Methode des ballistischen Transfers den Vorteil, daß hier die getroffenen Zellen auf einfache Weise von den nicht getroffenen abgetrennt werden können (DE-A-44 16 784.9). Es zeigt sich jedoch, daß bei der praktischen Anwendung der Zelltransfektion in der Humantherapie, wie bei einer Reihe anderer Problemstellungen, die Zahl der zu transfizierenden Zellen in vielen Fällen um mindestens eine Größenordnung größer ist. Abhängig von der Transfektionsrate muß davon ausgegangen werden, daß 10⁷ - 10⁸ Zellen pro Behandlungsschritt transfiziert werden müssen. Bei der bisher erreichbaren Zahl an transfizierbaren Zellen pro Arbeitsgang ergibt dies bis zu über hundert einzelne Transfektionsvorgänge ("Schüsse") pro Behandlung.

Diese Situation ist im Rahmen einer praktischen Anwendung der Methode in der Klinik nicht tragbar.

Die DE 36 02 949 A1 beschreibt letztlich ein Tele-Injektionsgerät zur Fernbehandlung von Tieren mit Injektionsspritzen, in dem eine Druckgaszuführung mit der Abschlusseinheit über ein Druckverteilungsstück in Verbindung steht. Eine solche Vorrichtung dient zum Schiessen von makroskaligen Projektilen.

Ausgehend von diesem Stand der Technik ist es Aufgabe der vorliegenden Erfindung, eine entsprechende Apparatur zu schaffen, mit der es möglich ist, eine größere Zahl von Zellen pro Schuß zu treffen.

Dabei stellen sich allerdings Probleme: Die Garbe der Mikroprojektile hat, bedingt durch die Konstruktion der Apparatur, eine gewisse Maximalgröße, die nicht überschritten werden kann. Mit wachsendem Abstand zwischen Zielzellen und Makroprojektil-Rückhaltung verbreitert sich zwar die Geschoßgarbe etwas, doch nimmt gleichzeitig die Geschwindigkeit, und damit die kinetische Energie der Mikroprojektile ab, die diese zum Eindringen in die Zelle benötigen.

Gleichzeitig läßt sich die Dichte der Zellen im Bereich der Geschoßgarbe nicht beliebig erhöhen. Die höchste Dichte ist in einer dichten Zelleinzelschicht erreicht, denn die Zellen wachsen nur in Ausnahmefällen übereinander, und es nimmt in diesem Falle auch die Treffeffizienz stark ab.

Der Vergrößerung der Makroprojektile steht entgegen, daß dadurch eher die Dichte der eintreffenden Geschoßgarbe in ihrem Zentrum erhöht, als ihre Fläche nennenswert vergrößert würde.

### Erfindungsgemäße technische Lösung

Gelöst wird die Aufgabe durch die kennzeichnenden Merkmale des Anspruchs 1.

Erfindungsgemäß wird die in einem Arbeitsgang, d.h. einer Druckgas-Stoßwelle, von Mikroprojektilen getroffene Fläche dadurch vergrößert, daß an Stelle des herkömmlichen Kopfstückes der von Bio-Rad vertriebenen Apparatur ein Kopfstück eingesetzt wird, das die bei der Gasentladung entstehende Druckwelle auf mehrere Makroprojektil-Halterungen verteilt, so daß eine Mehrzahl von Geschoßgarben entsteht, die sich im äußeren Bereich geringer Mikroprojektildichte überlagern. Das Druckverteilungsstück verteilt dabei den Druck über sieben Leitungen, sechs hexagonal angeordnete und eine zentrale Leitung, auf eine darunter liegende Platte mit Öffnungen für ebenso viele Makroprojektil-Einheiten der gleichen Anordnung. Bei der erfindungsgemäßen Anordnung von sieben Makroprojektil-Halterungen in einem Kopfstück lassen sich daher pro Schuss 10⁷ Zellen auf einer Petrischale von 10 cm Durchmesser behandeln.

Das dabei verwendete Kopfstück mit in ihm enthaltener Berstscheiben-Halterung und Druckverteilung, sowie die Platte mit Abstandsring und Scheibe zur Aufnahme des Einsatzstückes und der Ringplatte zur Makroprojektil-Halterung und -Rückhaltung sind Gegenstand des vorliegenden Patents.

Das Kopfstück setzt sich zusammen aus einem Flansch **1** und einem Druckverteilungsstück **2** mit daraus abgehenden Leitungen **3**, die durch eine Platte 4 in ihrer Position fixiert werden. Das Kopfstück bildet eine konstruktive Einheit, die während des Gebrauchs nicht demontiert wird. Alle Teile sind aus rostfreiem Edelstahl hergestellt und wahlweise miteinander verlötet oder durch Klebung verbunden.

Der Flansch **1** des Kopfstückes nimmt die Berstscheibe **5** auf und wird auf ein im Schiessaggregat befindliches Rohrgewinde geschraubt, das das Kopfstück mit der Helium-Druckleitung verbindet.

Die Rundscheibe **6** zur Aufnahme der Makroprojektilhalterungen wird über den Abstandsring **7** in der Platte **8** fixiert. Optional kann der Abstand zwischen Platte und Rundscheibe über ein Gewinde zwischen Platte und Abstandsring justiert werden, was die Feineinstellung der Partikelenergie beim Auftreffen auf die Zielzellen ermöglicht.

In die Rundscheibe werden die Einsatzstücke **9** eingesteckt, die das Gitter **10** zur Rückhaltung des Makroprojektils aufnehmen. Darauf liegen die Ringplatten **11**. Diese nehmen das Makroprojektil **12** auf. Dazu werden die Makroprojektile mit einem zylindrischen Körper in die Aussparung in der Ringplatte gedrückt.

Um eine Feinregulierung der auf die einzelnen Ausgänge entfallenden Druckanteile herbeizuführen, ist in einer oder mehrerer der Druckleitungen ein Reduzierventil eingebaut. Ferner kann der Abstand zwischen Zielzellen und Makroprojektil-Halterungen durch einen Abstandsring, der die Platte mit den Makroprojektil-Halterungen mit der Befestigung im Gehäuse verbindet, durch ein Gewinde feinreguliert werden.

### Beispiel:

Das Kopfstück wird nach Einlegen einer Berstscheibe (10,7 MPa (= 1550 psi)) auf das Gewinde des Schiessaggregats "Biolistic PDS -1000/HE" He" der Firma Bio-Rad (Hercules, California, USA) geschraubt und mit dem von der Herstellerfirma Bio-Rad gelieferten Drehmomentschlüssel festgezogen.

Das Schiessaggregat enthält sechs Schubrillen in der als "untere Vakuumkammer" bezeichneten Sektion. Die Platte zum Abstellen der Petrischale mit Zielzellen wird in eine der beiden untersten Schubrillen gesteckt.

Die Platte mit Abstandsring und Rundscheibe zur Aufnahme der Makroprojektile wird in die dritte Schubrille von unten gesteckt. Die Scheibe wird so verschoben, daß die sechs äußeren Einsatzstücke direkt unter den Auslaßrohren des Kopfstückes liegen.

Je 30 *µ*l einer Suspension von Goldpartikeln (1.6 *µ*m Durchmesser, bezogen von Bio-Rad, Hercules, CA, USA, Konzentration der Suspension: 60 mg/ml) werden auf insgesamt sieben Makrocarrier-Polymerplatten (Fa. Bio-Rad) aufpipettiert. Man wartet, bis sich das Gold abgesetzt hat, und zieht den Überstand vorsichtig ab.

Auf die benetzte Fläche wird 40 *µ*l einer 3+1 Mischung aus einer wässrigen DNS-Lösung (Konz.: 50 *µ*g fluoresceinmarkierter Oligodesoxyribo-Nucleotide/ml ) und einer Suspension kolloidaler paramagnetischer Partikel (mittlerer Durchmesser: 65 nm; Miltenyi GmbH, Bergisch-Gladbach; eingesetzt, wie vom Hersteller geliefert; Konz. unbekannt. Es kann vorteilhaft sein, die Magnetpartikel vor der Verwendung gegen Wasser zu dialysieren, um im Lagerungspuffer enthaltenes Natriumazid zu entfernen.) pipettiert. Man wirbelt das abgesetzte Gold auf und läßt das Gemisch sich absetzen. Man zieht die überstehende Flüssigkeit ab und läßt die Goldpartikel antrocknen.

10⁷ Zellen (Erythroleukämiezellinie K 562) werden in 10 ml RPMI-1640-Medium (10% fötales Kälberserum, FKS) auf die Petrischale gebracht und über Nacht im Brutschrank inkubiert. Direkt vor der Transfektion wird das Medium abgesaugt, die Zellen werden einmal mit PBS-Medium gespült, trockengesaugt und transfiziert.

Der ballistische Transfer wird nach Vorschrift des Herstellers des Apparats durchgeführt. Die verwendete Berstscheibe entspricht einem Sollbruchdruck von 10,7 MPa (= 1550 psi). Der Druck der Vakuumzelle beträgt 508 mm Quecksilbersäule (20 inches Hg). Für die anschließende magnetische Sortierung wird auf die DE-A-44 16 784.9 verwiesen.

### Kurze Erläuterung der Zeichnungen:

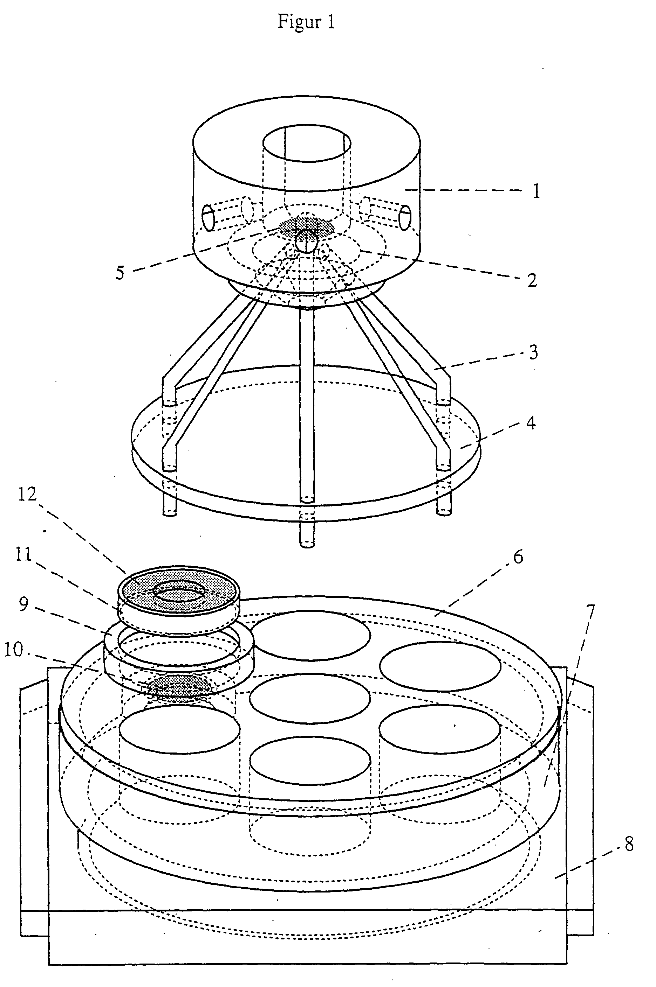

Figur 1 zeigt eine Gesamtansicht aller Teile im zusammengesteckten Zustand. Zur größeren Klarheit ist nur ein Einsatzstück gezeichnet.

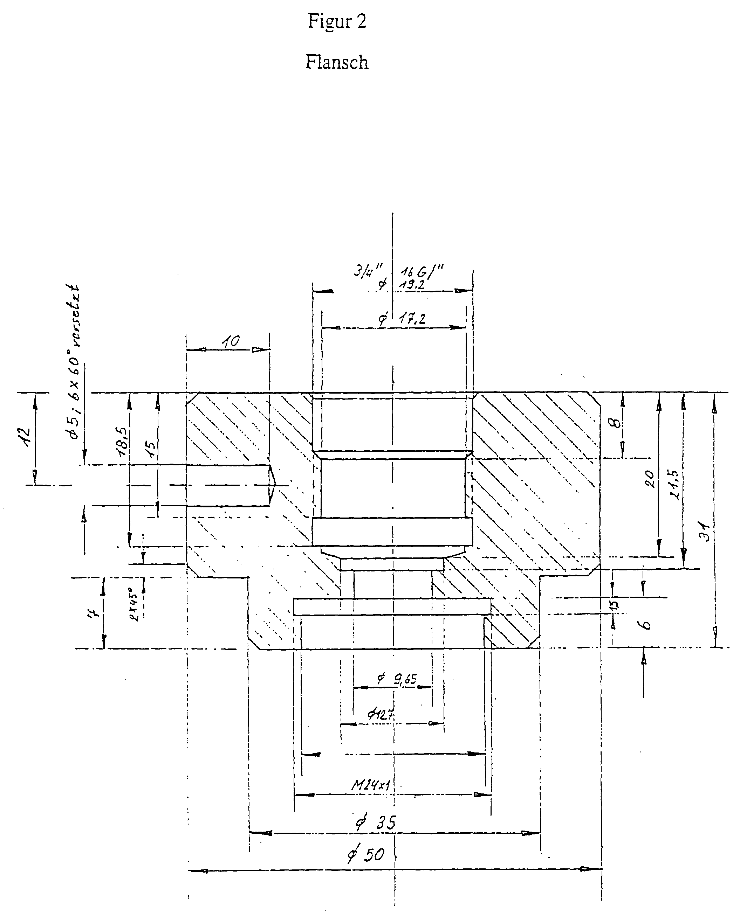

Figur 2 zeigt einen Schnitt des Flansches.

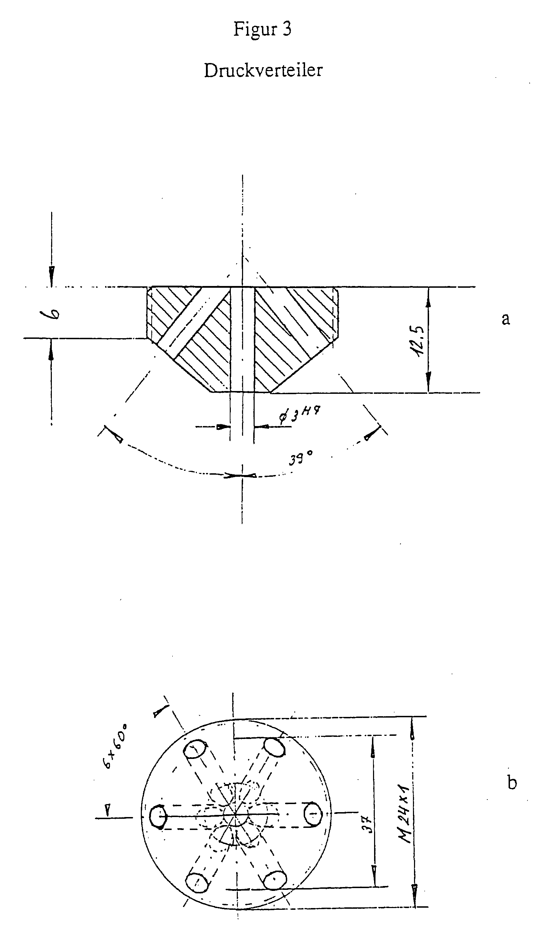

Figur 3a zeigt einen Schnitt, Figur 3b eine Aufsicht des Verteilerringes.

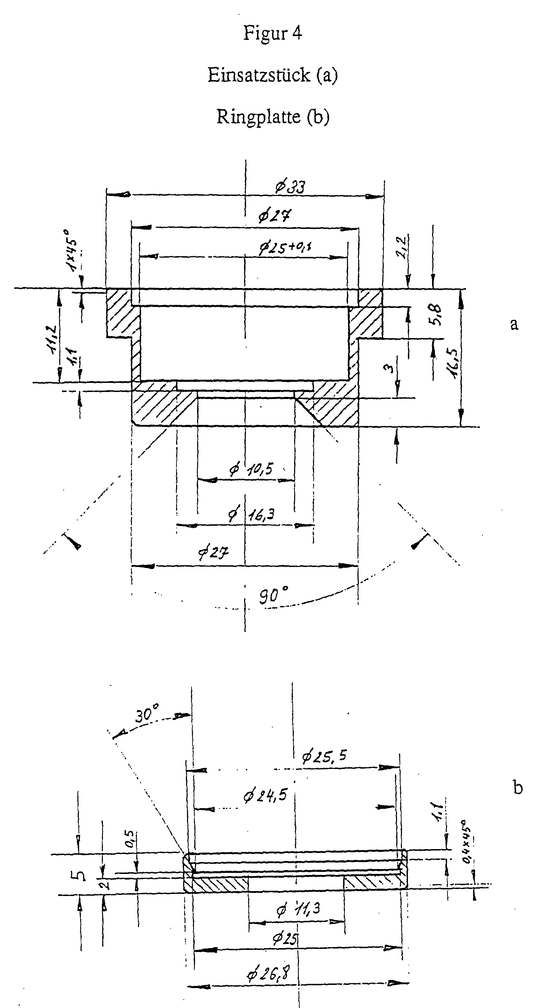

Figur 4a zeigt das Einsatzstück, Figur 4b die Ringplatte in umgekehrter Orientierung.

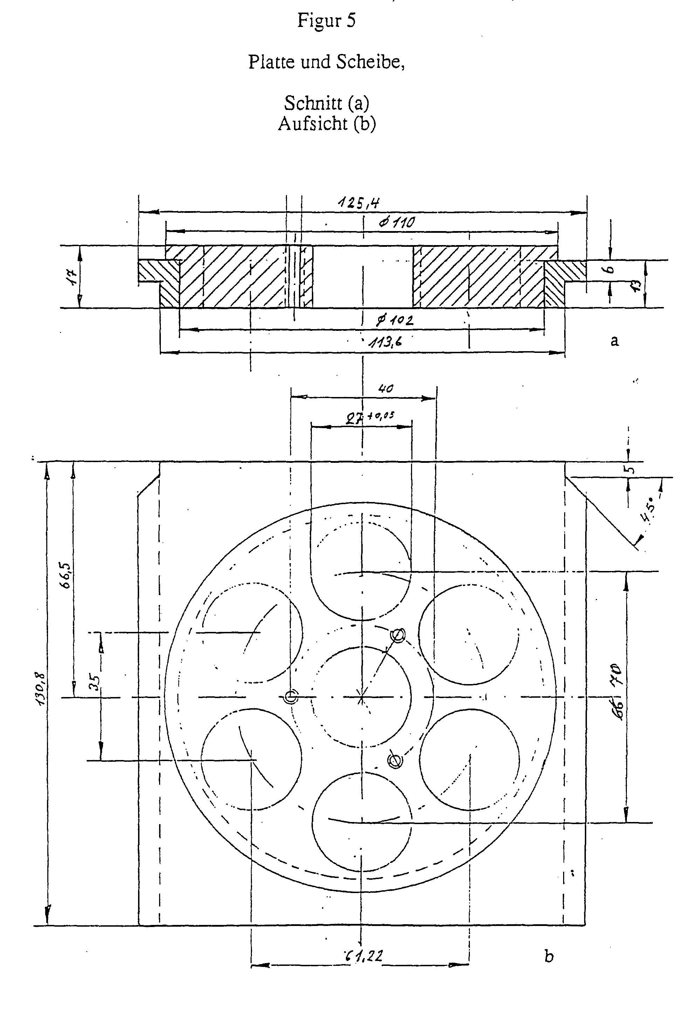

Figur 5 a zeigt einen Schnitt von Platte und Scheibe, Figur 5 b die dazu gehörige Aufsicht.

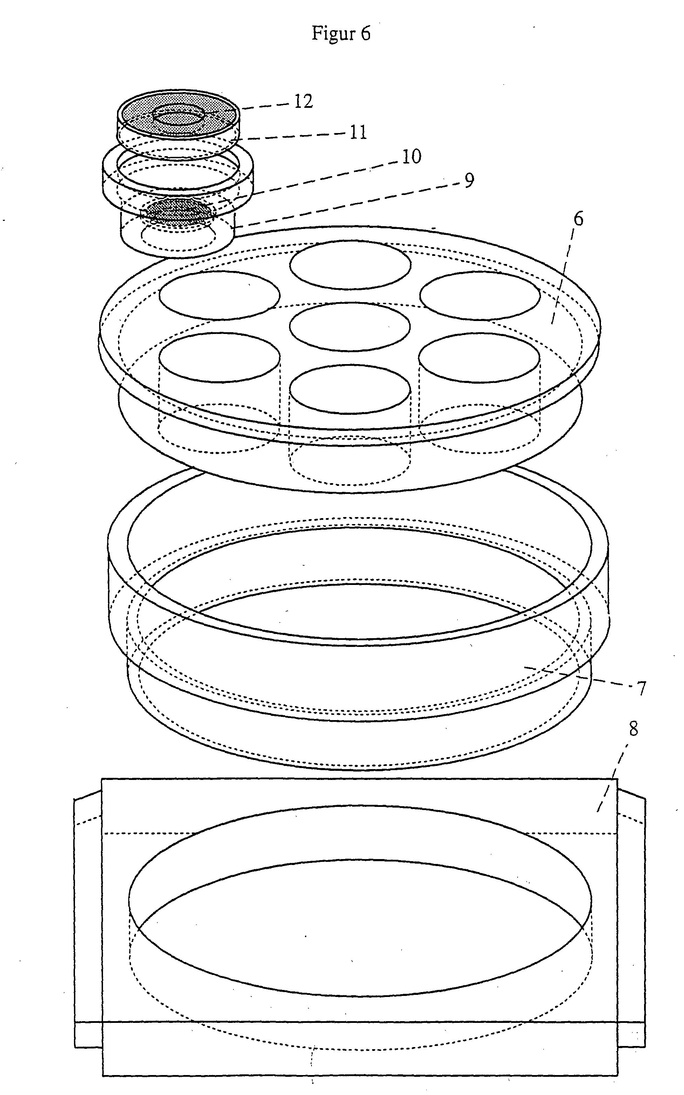

Figur 6 zeigt eine Explosionszeichnung von Platte, Abstandsring, Scheibe und Einsatzstück mit Ringplatte.

Alle Maße, wenn nicht anders gekennzeichnet, verstehen sich als Millimeterangaben

### Bezugszeichen

- 1: Flansch
- 2: Druckverteilungsstück
- 3: Abgehende Leitungen
- 4: Platte
- 5: Berstscheibe
- 6: Rundscheibe
- 7: Abstandsring
- 8: Platte
- 9: Einsatzstücke
- 10: Gitter
- 11: Ringplatte
- 12: Makroprojektil

## Patentansprüche

1. Vorrichtung zur Druckverteilung im Kopfstück einer Apparatur zum ballistischen Transfer von Zellen, **dadurch gekennzeichnet,**
• **daß** das Kopfstück eine konstruktive Einheit bildet, bestehend aus einem Flansch (1) und einem Druckverteilungsstück (2) mit daraus abgehenden Leitungen (3), die durch eine Platte (4) in ihrer Position fixiert sind, und wobei
• der nach Bruch der Berstscheibe (5) in die Vorrichtung strömende Druck über ein Druckverteilungsstück (2) auf eine Mehrzahl von Makroprojektil-Halterungen geleitet wird, in denen je ein Makroprojektil (12) in Richtung der Zielzellen beschleunigt wird, wobei die am Makroprojektil (12) haftenden Mikroprojektile nach Rückhaltung desselben auf die Zielzellen weiterfliegen und diese treffen.

2. Vorrichtung nach Anspruch 1, bei der das Druckverteilungsstück (2) den Druck über sieben Leitungen (3), sechs hexagonal angeordnete und eine zentrale Leitung, auf eine darunter liegende Platte (8) mit Öffnungen für ebenso viele Makroprojektil-Einheiten der gleichen Anordnung verteilt.

3. Vorrichtung nach Anspruch 1 oder 2, wobei in einer oder mehrerer der Druckleitungen (3) ein Reduzierventil eingebaut ist, um eine Feinregulierung der auf die einzelnen Ausgänge entfallenden Druckanteile herbeizuführen.

4. Vorrichtung nach Anspruch 1 oder 2, wobei der Abstand zwischen Zielzellen und Makroprojektil-Halterungen durch einen Abstandsring (7), der die Platte (8) mit den Makroprojektil-Halterungen mit der Befestigung im Gehäuse verbindet, durch ein Gewinde feinreguliert werden kann.

## Claims

1. Device for distributing the pressure in the head of an apparatus to produce the ballistic transfer of cells, **characterised by** the fact that
• the head forms a single structural unit, consisting of a flange (1) and a pressure distribution piece (2), to which lines (3) are connected, the positions of which are fixed by a plate (4), and in which,
• the pressure flowing into the device following the rupture of a disk (5) is fed through a pressure distribution piece (2) onto a number of macro-projectile mountings, in each of which a macro-projectile (12) is accelerated in the direction of the target cells, and in which, after the macro-projectile (12) is restrained, the micro-projectiles attached to it continue towards the target cells and strike them.

2. Device in accordance with Claim 1, in which the pressure distribution piece (2) distributes the pressure through seven lines (3), six of them arranged in a hexagon around a central line, onto a plate below (8) with apertures for an equal number of macro-projectile units in the same arrangement.

3. Device in accordance with Claim 1 or 2, in which a reducing valve is fitted into one or more of the pressure lines (3), in order to achieve fine regulation of the proportion of the pressure at the individual exits

4. Device in accordance with Claim 1 or 2, in which the fine adjustment can be made, by means of a thread, of the distance between the target cells and macro-projectile mountings, using a spacer ring (7), which joins the plate (8) containing the macro-projectile mountings to the attachment in the housing.

## Revendications

1. Dispositif de répartition de la pression dans l'embout d'un système de transfert balistique de cellules **caractérisé par le fait**
• **que** l'embout constitue une construction homogène, composée d'une bride (1) et d'un élément de répartition de la pression (2) d'où partent des canalisations (3) maintenues en place par une plaque (4) et compte tenu du fait que
• la pression arrivant dans le dispositif suite à la rupture du disque de rupture (5) est dirigée à l'aide d'un élément de répartition de la pression (2) sur plusieurs supports de macroprojectiles, chaque support contenant un macroprojectile (12) qui est propulsé en direction des cellules-cibles, les microprojectiles fixés au macroprojectile (12) continuant, une fois ce dernier arrêté, leur trajectoire en direction des cellules-cibles et les frappant.

2. Dispositif selon la revendication n° 1, dans lequel l'élément de répartition de la pression (2) répartit la pression au moyen de sept canalisations (3), six disposées hexagonalement et une canalisation centrale, sur une plaque (8) située au-dessous percées d'ouvertures pour un nombre d'unités de macroprojectiles équivalant à la même disposition.

3. Dispositif selon la revendication n° 1 ou 2, un détendeur étant incorporé dans une ou plusieurs des canalisations sous pression (3) afin d'obtenir un réglage de précision des quantités de pression correspondant aux différentes sorties.

4. Dispositif selon la revendication n° 1 ou 2, la distance entre les cellules-cibles et les supports de macroprojectiles pouvant être réglée avec précision grâce à une bague d'écartement (7) qui relie la plaque (8) portant les supports de macroprojectiles à la fixation située dans le boîtier et qui se manoeuvre à l'aide d'une vis.
